# EUROPEAN PATENT SPECIFICATION

(11) **EP 2 393 536 B1**
(45) Date of publication and mention of the grant of the patent: **19.04.2017**
(21) Application number: 10738854.8
(22) Date of filing: 04.02.2010
(51) Int. Cl.: A61M 5/32

(54) **PEN NEEDLE ASSEMBLY HAVING BIODEGRADABLE COMPONENTS**
STIFTNADELANORDNUNG MIT BIOLOGISCH ABBAUBAREN KOMPONENTEN
ENSEMBLE AIGUILLE DE STYLET À ÉLÉMENTS BIODÉGRADABLES

(30) Priority: 06.02.2009 US 150681 P
(43) Date of publication of application: 14.12.2011
(73) Proprietor: Becton, Dickinson and Company, Franklin Lakes, NJ 07417-1880 (US)
(72) Inventor: HORVATH, Joshua, Sparta New Jersey 07871 (US); BRIZZOLARA, Joseph P., Vernon New Jersey 07419 (US); ROSEN, Edward, Morristown New Jersey 07960 (US)
(74) Representative: dompatent von Kreisler Selting Werner - Partnerschaft von Patent- und Rechtsanwälten mbB
(86) International application number: PCT/US2010/000304
(87) International publication number: WO 2010/090734

(56) References cited:
- EP-A1- 1 138 338
- EP-A1- 1 346 739
- WO-A1-03/045480
- WO-A2-2006/071813
- US-A- 3 073 307
- US-A- 4 300 678
- US-A1- 2002 183 696
- US-A1- 2007 225 676
- US-A1- 2008 077 096
- US-B1- 6 478 780

## Description

### Cross Reference to Related Application

### Field of the Invention

The present invention relates generally to a biodegradable needle assembly for a drug delivery device. More particularly, the present invention relates to a pen needle having components made from a biodegradable polymer material. Still more particularly, the present invention relates to a pen needle having components made of polylactide (PLA), polyvinyl alcohol or starch-filled polypropylene materials, thereby providing a more environmentally friendly drug delivery needle for a drug delivery device.

### Background of the Invention

Insulin and other injectable medications are commonly given with drug delivery pens, whereby a disposable pen needle is attached to facilitate drug container access and allow fluid egress from the container through the needle into the patient.

As technology and competition advance, driving the desire for shorter, thinner, less painful, and more efficacious injections, the design of the pen needle and parts thereof becomes more and more important. Designs need to proactively address ergonomically improving injection technique, injection depth control and accuracy, the ability to be safely used and transported to disposal, and protection against misuse while maintaining the ability to be economically manufactured on a mass production scale. Increasingly, environmental factors relating to the manufacture and disposal of pen needles must also be considered.

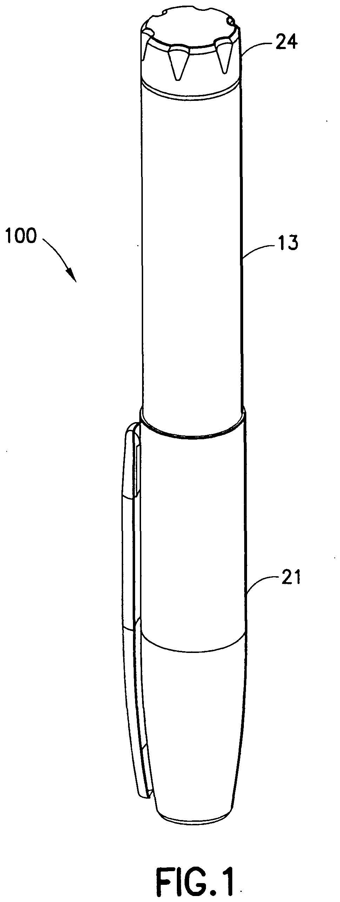

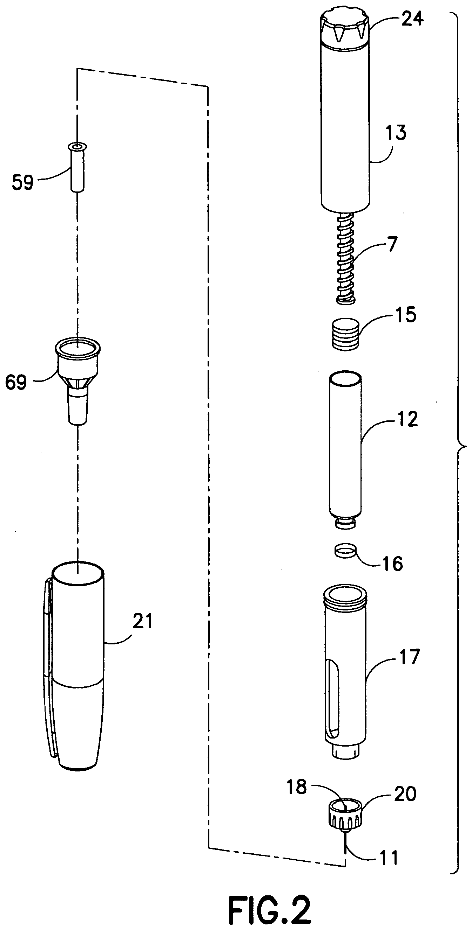

Drug delivery devices, such as the exemplary drug delivery pen 100 shown in FIGS. 1 and 2, can be designed for subcutaneous, as well as intradermal, injections and typically comprise a dose knob/button 24, an outer sleeve 13, and a cap 21. The dose knob/button 24 allows a user to set the dosage of medication to be injected. The outer sleeve 13 is gripped by the user when injecting medication. The cap 21 is used by the user to securely hold the drug delivery pen 100 in a shirt pocket, purse or other suitable location and provide cover/protection from accidental needle injury.

FIG. 2 is an exploded view of the drug delivery pen 100 of FIG. 1. The dose knob/button 24 has a dual purpose and is used both to set the dosage of the medication to be injected and to inject the dosed medicament via the leadscrew 7 and stopper 15 through the medicament cartridge 12, which is attached to the drug delivery pen through a lower housing 17. In standard drug delivery pens, the dosing and delivery mechanisms are all found within the outer sleeve 13 and are not described in greater detail here as they are understood by those knowledgeable of the prior art. The distal movement of the plunger or stopper 15 within the medicament cartridge 12 causes medication to be forced into the steel patient needle 11 of the hub 20. The medicament cartridge 12 is sealed by septum 16, which is punctured by a steel septum penetrating needle cannula 18 located within the hub 20. The hub 20 is preferably screwed onto the lower housing 17, although other attachment means can be used, such as attaching to the cartridge. To protect a user, or anyone who handles the pen injection device 100, an outer cover 69, which attaches to the hub 20, covers the hub. An inner shield 59 covers the patient needle 11 within the outer cover 69. The inner shield 59 can be secured to the hub 20 to cover the patient needle by any suitable means, such as an interference fit or a snap fit. The outer cover 69 and the inner shield 59 are removed prior to use. The cap 21 fits snugly against outer sleeve 13 to allow a user to securely carry the drug delivery pen 100.

The medicament cartridge 12 is typically a glass tube sealed at one end with the septum 16 and sealed at the other end with the stopper 15. The septum 16 is pierceable by a septum penetrating cannula 18 in the hub 20, but does not move with respect to the medicament cartridge 12. The stopper 15 is axially displaceable within the medicament cartridge 12 while maintaining a fluid tight seal.

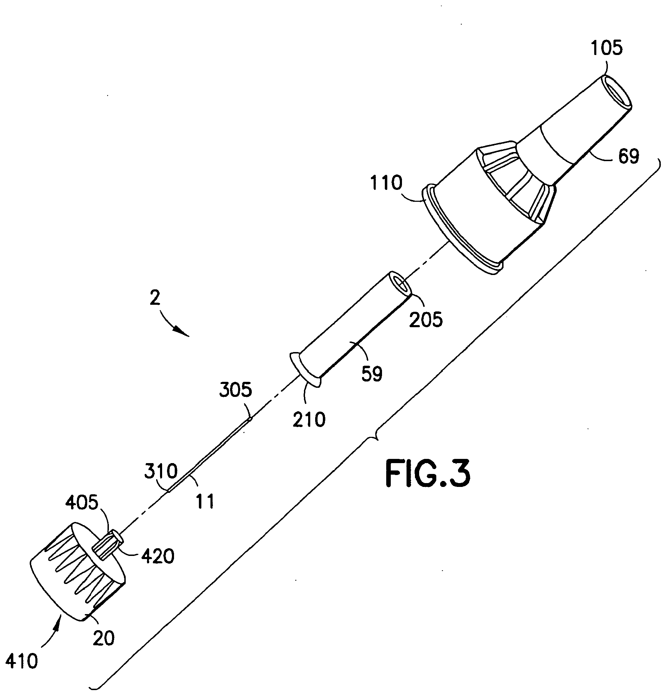

An exploded perspective view of a pen needle 2 of an exemplary drug delivery pen is shown in FIG. 3. The pen needle 2 includes the cover (outer shield) 69, an inner shield 59, a needle cannula 11, and a hub 20. A proximal end 310 of the needle cannula 11 is inserted into a center opening in the distal (patient) end 405 of the hub 20 until a predetermined length of the distal (patient) end 305 of the needle cannula 11 remains extended. The needle cannula 11 is secured by epoxy or adhesive in the distal end 405 of the hub 20 within the hub protrusion 420.

To protect users from injury and the needle cannula 11 from being damaged, the inner shield 59 covers the exposed portion of the needle cannula 11. The open proximal end 210 of the inner shield 59 is placed over the exposed portion of the needle cannula 11. The open proximal end 110 of the cover 69 envelops the inner shield 59, needle cannula 11, and hub 20.

The distal end 105 of the cover 69 is closed to prevent contamination and damage to the inner components of pen needle 2, and to prevent injury to anyone who may handle it prior to use. The proximal end 410 of the hub 20 is typically covered by a sanitary paper or foil cover (not shown) glued on an end 110 of the cover 69. The drug delivery pen is then ready for shipment to a user. When the user is ready to use the drug delivery pen, the sanitary cover (not shown) is removed from the cover 69, the hub 20 is screwed onto a lower housing 17 of a standard pen 100 (FIGS. 1 and 2), and the cover 69 and shield 59 are separately removed from the hub 20/cannula 11 subassembly by a pulling action. The distal end 205 of the inner shield 59 is closed to cover the distal end 305 of the needle cannula 11 after the cover 69 is removed to protect the user from an accidental stick. The inner shield 59 is then removed to access the needle cannula 11. Thus, two separate pulling actions are required to remove both the cover 69 and the shield 59.

Existing pen needles include components made of petroleum-based polymers for packaging, including the outer cover, inner shield and parts of the label tab. Petroleum based polymers do not degrade in landfills. Thus, a need exists for an environmentally friendly pen needle.

The rate limiting factor on existing pen needle lines is feeding of the label tab material. Syringe assembly lines often run at twice the rate of the pen needle lines. Thus, a need also exists for a pen needle in which the paper label tab is eliminated, thereby increasing the production speed.

Existing drug delivery pens are disclosed in U.S. Patent Application Publication Nos. 2006/0229562 to Marsh et al. and 2007/0149924 to R. Marsh, Patent Application Publications Nr. EP 1 346 739 A1, EP 1 138 338 A1 and WO 2007/047403 A1 disclose prior art needle hubs with needle covers.

### Summary of the Invention

The invention is defined in claim 1. In accordance with an aspect of the present invention, a pen needle or other drug delivery needle has biodegradable components, thereby providing an environmentally friendly needle.

In accordance with another aspect of the present invention, a pen needle includes a cap made of a biodegradable material, thereby increasing the production rate as well as providing an environmentally friendly pen needle.

In accordance with another aspect of the present invention, an outer cover of a pen needle is made with less material.

The foregoing objects are basically attained by providing a drug delivery needle assembly including a hub and a needle fixedly connected to the hub. A cover member removably receives the hub. A sealing member is removably connected to the cover member. At least one of the hub, the cover member and the sealing member is made of a biodegradable material, thereby providing a more environmentally friendly drug delivery needle.

Objects, advantages, and salient features of the invention will become apparent from the following detailed description, which, taken in conjunction with the annexed drawings, discloses exemplary embodiments of the invention.

### Brief Description of the Drawings

The above benefits and other advantages of the various embodiments of the present invention will be more apparent from the following detailed description of exemplary embodiments of the present invention and from the accompanying drawing figures, in which:
FIG. 1 is a perspective view of an assembled drug delivery pen;
FIG. 2 is an exploded perspective view of the drug delivery pen of FIG. 1;
FIG. 3 is an exploded perspective view of a needle of the drug delivery pen of FIG. 1;
FIG. 4 is an exploded perspective view of a pen needle assembly according to an exemplary embodiment of the present invention including an inner shield and a cap connected to a needle hub;
FIG. 5 is an exploded perspective view of a pen needle assembly not according to the present invention including an inner shield and a label connected to a needle hub;
FIG. 6 is an exploded perspective view of a pen needle assembly not according to the present invention including an outer cover and a cap connected to a needle hub; and
FIG. 7 is a plot of peak penetration force vs. outer cover material for a needle of a pen needle to penetrate the outer wall of the outer cover.

Throughout the drawings, like reference numbers will be understood to refer to like parts, components and structures.

### Detailed Description of the Exemplary Embodiments

The following description and details of exemplary embodiments of the present invention, while generally disclosed in a typical drug delivery pen, as shown in FIGS. 1 - 3, could more broadly apply to a needle for use in conjunction with, or incorporated onto, other injection devices, such as syringes, autoinjectors and infusion devices.

Components of a pen needle assembly according to exemplary embodiments of the present invention are made of a biodegradable polymer, such as polylactide (PLA), which is made from corn and decomposes in landfills. PLA polymers are substantially stronger than polypropylene, which is currently used to manufacture components of pen needles, thereby providing increased resistance to penetration with a similar thickness. Thus, components made of PLA polymers may be made with a reduced wall thickness while still providing increased resistance to penetration than polypropylene components. The PLA polymer may be used to manufacture any component of a drug delivery pen that is not in a fluid delivery path, such as, but not limited to, pen needle hubs, syringe caps (both plunger and patient ends), sharps disposal containers, lancet bodies and caps, non-fluid path components in safety syringes, non-fluid path components in regular syringes and external packaging. Alternative environmentally-friendly materials for manufacturing components of a pen needle include polyvinyl alcohol, a degradable polymer that dissolves when left in contact with water, and starch-filled polypropylene, which has a reduced petroleum-based polymer content.

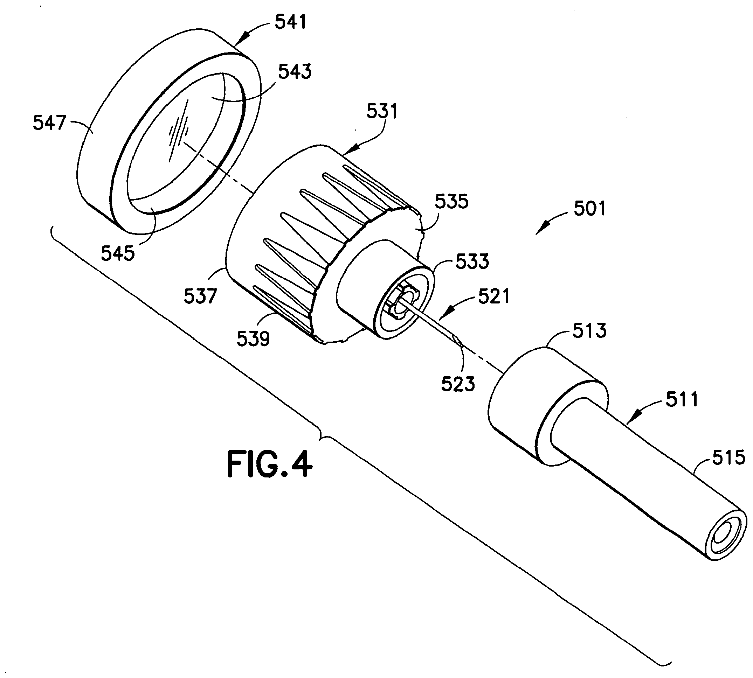

In an exemplary embodiment of the present invention shown in FIG. 4, a pen needle assembly 501 includes an inner shield 511, a steel needle 521, a hub 531 and a cap 541. The inner shield 511 is disposed on a first end 533 of the hub 531 such that an end 513 of the inner shield abuts the base 535 of the hub. The patient end 523 of the needle 521 is received by the projection 515 of the inner shield.

The cap 541 is disposed on a second end 537 of the hub 531 such that the second end 537 of the hub abuts an inner surface 543 of the cap. The cap 541 protects the non-patient end of the needle 521. The side walls 539 of the hub 531 are exposed when the inner shield 511 and cap 541 are disposed on the hub. Sterility of the needle 521 is preserved by the inner shield 511 at a first end 533 of the hub 531 and the cap 541 at the second end 537 of the hub. Therefore, neither an outer cover nor a paper or foil label tab is needed.

The cap 541 may be disposed over the outside of the hub 531 or disposed within the hub, thereby providing a sterile seal between the cap and the hub. The second end 537 of the hub 531 may abut the inner surface 543 of the cap 541 such that a portion of the outer surface of the side wall 539 is disposed adjacent the inner surface 545 of the cap 541. Alternatively, the outer surface 547 of the cap 541 may be disposed within the hub 531.

In the exemplary embodiment of FIG. 4, the inner shield 511, the hub 531 and/or the cap 541 may be made of a biodegradable material.

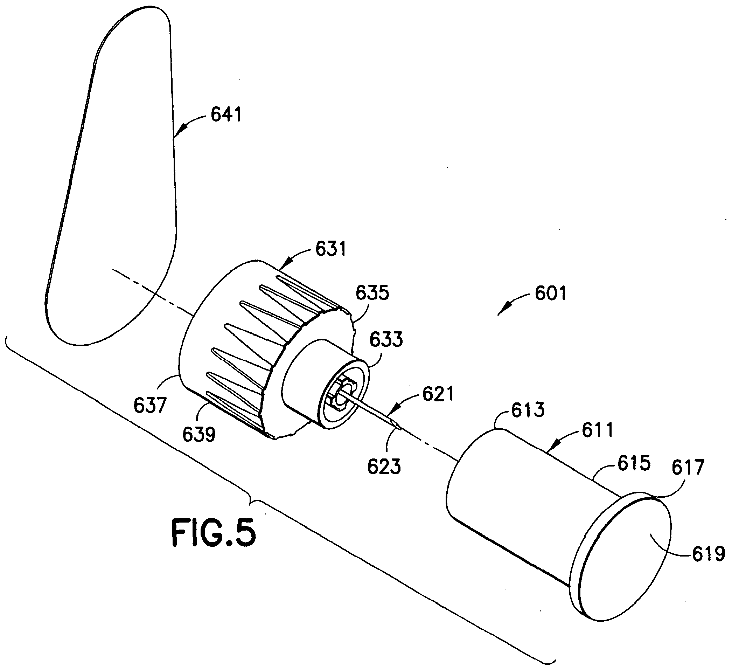

In another exemplary embodiment not according to the present invention shown in FIG. 5, a pen needle assembly 601 includes an inner shield 611, a steel needle 621, a hub 631 and a label tab 641. The inner shield 611 is disposed on a first end 633 of the hub 631 such that an end 613 of the inner shield abuts the base 635 of the hub. The patient end 623 of the needle 621 is received within the body 615 of the inner shield 611. A flange 617 may be disposed at an opposite end 619 of the inner shield 611 to facilitate manipulation of the inner shield.

The label tab 641 is bonded to a second end 637 of the hub 631. The label tab 641 protects the non-patient end of the needle 621. The side walls 639 of the hub 631 are exposed when the inner shield 611 and label tab 641 are disposed on the hub. Sterility of the needle 621 is preserved by the inner shield 611 at a first end 633 of the hub 631 and the label tab 641 at the second end 637 of the hub. As in the previous embodiment, no outer cover is needed.

In the exemplary embodiment of FIG. 5, the inner shield 611 and/or the hub 631 may be made of a biodegradable material.

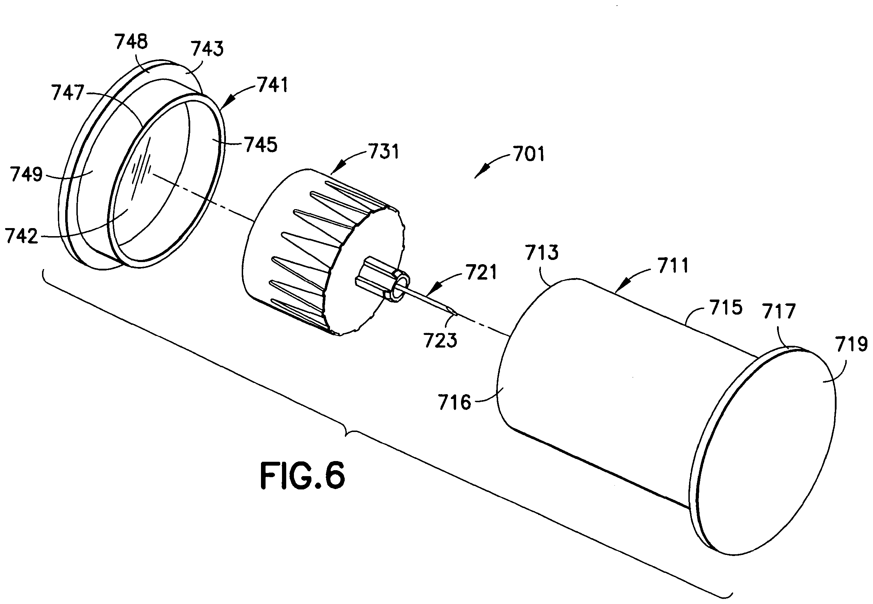

In another exemplary embodiment not according to the present invention shown in FIG. 6, a pen needle assembly 701 includes an outer cover 711, a steel needle 721, a hub 731 and a cap 741. The hub 731 and needle 721 are disposed within the outer cover 711 and cap 741, such that an end 713 of the outer cover 711 abuts the base 743 of the cap. The patient end 723 of the needle 721 is received by the body 715 of the outer cover 711, and the cap 741 covers the non-patient end of the needle 721. A flange 717 may be disposed at an opposite end 719 of the outer cover 711 to facilitate manipulation of the inner shield. Accordingly, the hub 731 and needle 721 are completely enclosed within the outer cover 711 and cap 741.

The cap 741 is preferably disposed over the outside of the outer cover 711. The end 713 of the outer cover 711 abuts an inner portion 742 of the base 743 of the cap 741 within a wall 747 such that at least a portion of the outer surface 716 of the body 715 of the outer cover 711 is disposed adjacent to an inner surface 745 of the wall 747 of the cap 741. Alternatively, the cap may be disposed within the outer cover 711, which would require a longer outer cover (and therefore more polymer to manufacture the cover) to provide clearance with the hub 731 disposed therein. The end 713 of the outer cover shield 711 abuts an outer portion 748 of the base 743 of the cap 741 such that at least a portion of an inner surface of the body 715 of the outer cover 711 is disposed adjacent to an outer surface 749 of the wall 747 of the cap 741.

In the exemplary embodiment of FIG. 6, the outer cover 711, the hub 731 and/or the cap 741 may be made of a biodegradable material.

Existing outer covers typically require 1.17 mL of resin compared to 0.85 mL of resin for outer cover 711 and cap 741 of the exemplary embodiment of FIG. 6. A typical wall thickness for a polypropylene outer cover for an existing drug delivery pen is approximately 0.050 inches. Typical wall thickness for a PLA outer cover of an exemplary embodiment of the present invention is approximately 0.025 inches.

Production rates of the pen needle assemblies may be increased by using caps, as shown in FIGS. 4 and 6, and eliminating the paper label tabs currently used to seal the non-patient needle end of the hub.

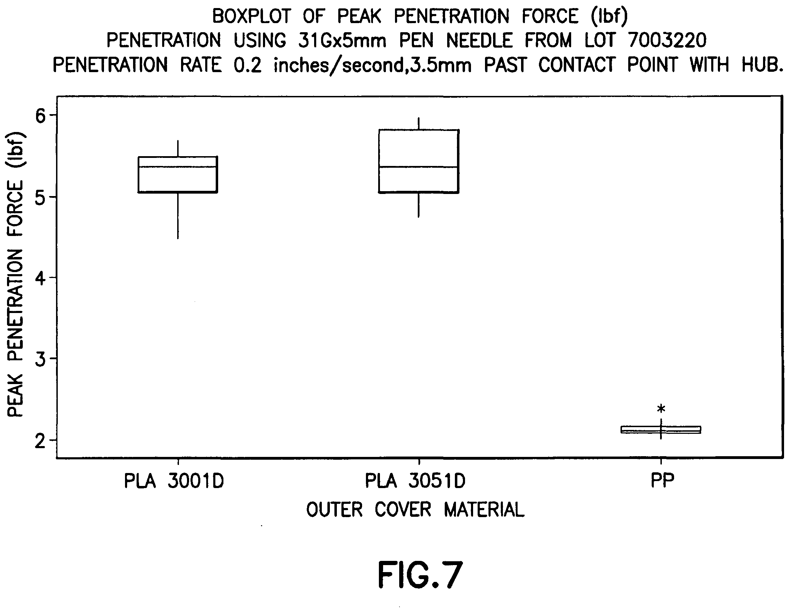

FIG. 7 is a plot of the peak penetration force required for a standard production 31 gage x 5 mm pen needle to penetrate the outer walls of an outer cover. Peak penetration force is plotted as a function of the outer cover material. A standard polypropylene outer cover has an average peak penetration force of 2.1 lbf. Outer covers made from PLA 3001D and PLA 3051, available from NatureWorks LLC of Minnetonka, Minnesota, have average peak penetration forces of 5.2 lbf and 5.4 lbf, respectively. Thus, as shown in FIG. 7, a greater force is required to penetrate the outer cover made of a PLA polymer than an outer cover made of polypropylene. Accordingly, outer covers and other components made of a PLA polymer can have a reduced wall thickness, while still providing equivalent or greater penetration resistance.

The foregoing embodiments and advantages are merely exemplary and are not to be construed as limiting the scope of the present invention. The description of an exemplary embodiment of the present invention is intended to be illustrative, and not to limit the scope of the present invention. Various modifications, alternatives and variations will be apparent to those of ordinary skill in the art, and are intended to fall within the scope of the invention as defined in the appended claims and their equivalents.

## Claims

1. A drug delivery needle assembly, comprising:
a hub (20, 531, 631) having side walls (539, 639) and a base (535);
a needle (11, 521, 621) fixedly connected to said hub;
an inner shield (511, 611) removably receiving said hub and covering a first end of said needle and
a cap (541, 641) removably connected to an end surface of said hub for covering a second end of said needle prior to using said needle and forming a sterile seal with said hub;
**characterized in that**
said inner shield and said hub form an interference fit;
said cap and at least one of said hub and said inner shield are made of a biodegradable material, and
said side walls of said hub (20, 531, 631) are exposed when said inner shield and said cap are connected to said hub, and
said inner shield is disposed on a first end (533, 633) of said hub such that an end (513, 613) of the inner shield abuts said base (535) of the hub and
a second end (537, 637) of the hub abuts an inner surface (545, 645) of the cap (541, 641) such that a portion of the outer surface of the side wall (539, 639) is disposed adjacent said inner surface (545, 645).

2. The drug delivery needle assembly according to claim 1, wherein
said biodegradable material comprises a biodegradable polymer selected from the group consisting of polylactide, polyvinyl alcohol, and starch-filled polypropylene.

3. The drug delivery needle according to claim 1, wherein
said inner shield has a wall thickness of approximately 0.025 inches.

4. The drug delivery needle according to claim 1, wherein said cap has a flange to facilitate manipulation thereof.

## Patentansprüche

1. Medikamentenausgabenadelanordnung mit:
einem Ansatz (20, 531, 631) mit Seitenwänden (539, 639) und einer Basis (535);
einer fest mit dem Ansatz verbundenen Nadel (11, 521, 621);
einem inneren Schutz (511, 611), der den Ansatz lösbar aufnimmt und ein erstes Ende der Nadel bedeckt, und
einer Kappe (541, 641), die lösbar mit einer Endfläche des Ansatzes verbunden ist, um vor dem Gebrauch der Nadel ein zweites Ende der Nadel zu bedecken und eine sterile Dichtung mit dem Ansatz zu bilden;
**dadurch gekennzeichnet, dass**
der innere Schutz und der Ansatz eine Presspassung bilden;
die Kappe und der Ansatz und/oder der innere Schutz aus biologisch abbaubarem Material bestehen, und
die Seitenwände des Ansatzes (20, 531, 631) freiliegen, wenn der innere Schutz und die Kappe mit dem Ansatz verbunden sind, und
der innere Schutz an einem ersten Ende (533, 633) des Ansatzes derart angeordnet ist, dass ein Ende (513, 613) des inneren Schutzes an der Basis (535) des Ansatzes anliegt, und
ein zweites Ende (537, 637) des Ansatzes an einer Innenfläche (545, 645) der Kappe (541, 641) derart anliegt, dass ein Bereich der Außenfläche der Seitenwand (539, 639) der Innenfläche (545, 645) benachbart angeordnet ist.

2. Medikamentenausgabenadelanordnung nach Anspruch 1, bei welcher das biologisch abbaubare Material ein biologisch abbaubares Polymer aufweist, das aus der Gruppe bestehend aus Polylactid, Polyvinylalkohol und mit Stärke gefülltem Polypropylen gewählt ist.

3. Medikamentenausgabenadelanordnung nach Anspruch 1, bei welcher der innere Schutz eine Wanddicke von ungefähr 0,025 Inch aufweist.

4. Medikamentenausgabenadelanordnung nach Anspruch 1, bei welcher die Kappe einen Flansch aufweist, um deren Handhabung zu vereinfachen.

## Revendications

1. Ensemble d'aiguille d'administration de médicament, comprenant :
un raccord (20, 531, 631) ayant des parois latérales (539, 639) et une base (535) ;
une aiguille (11, 521, 621) reliée de manière fixe audit raccord ;
un protecteur interne (511, 611) recevant de manière amovible ledit raccord et couvrant une première extrémité de ladite aiguille et
un capuchon (541, 641) relié de manière amovible à une surface d'extrémité dudit raccord pour couvrir une deuxième extrémité de ladite aiguille avant d'utiliser ladite aiguille et formant un joint stérile avec ledit raccord ;
**caractérisé en ce que**
ledit protecteur interne et ledit raccord forment un ajustement avec serrage ;
ledit capuchon et au moins l'un dudit raccord et dudit protecteur interne sont réalisés en un matériau biodégradable, et
lesdites parois latérales dudit raccord (20, 531, 631) sont exposées lorsque ledit protecteur interne et ledit capuchon sont reliés audit raccord, et
ledit protecteur interne est disposé sur une première extrémité (533, 633) dudit raccord de sorte qu'une extrémité (513, 613) du protecteur interne vient en butée contre ladite base (535) du raccord et
une deuxième extrémité (537, 637) du raccord vient en butée contre une surface interne (545, 645) du capuchon (541, 641) de sorte qu'une partie de la surface externe de la paroi latérale (539, 639) est disposée de manière adjacente à ladite surface interne (545, 645).

2. Ensemble d'aiguille d'administration de médicament selon la revendication 1, dans lequel
ledit matériau biodégradable comprend un polymère biodégradable choisi dans le groupe constitué de polylactide, d'alcool polyvinylique, et de polypropylène chargé en amidon.

3. Aiguille d'administration de médicament selon la revendication 1, dans laquelle
ledit protecteur interne a une épaisseur de paroi d'environ 0,025 pouce.

4. Aiguille d'administration de médicament selon la revendication 1, dans laquelle ledit capuchon a un rebord pour faciliter sa manipulation.
